(19) Europäisches Patentamt
European Patent Office
Office européen des brevets

(11) Publication number: 0 437 640 A1

# (12) EUROPEAN PATENT APPLICATION

(21) Application number: 90100565.2

(51) Int. Cl.5: C07C 327/42, A01N 37/46

(22) Date of filing: 12.01.90

(43) Date of publication of application:
24.07.91 Bulletin 91/30

(84) Designated Contracting States:
DE

(71) Applicant: SHELL INTERNATIONALE RESEARCH MAATSCHAPPIJ B.V.
Carel van Bylandtlaan 30
NL-2596 HR Den Haag(NL)

(72) Inventor: Buck, Wolfgang
In der Dörrweise 37
W-6507 Ingelheim am Rhein(DE)
Inventor: Raddatz, Erich
Carrera 1-Oe No.5265
Cali, Colombia(CO)

(74) Representative: Bennett, David Arthur Horder et al
4, York Road
London SE1 7NA(GB)

## (54) Fungicidal amino-thiocarboxylic acid amides.

(57) A compound according to general formula I

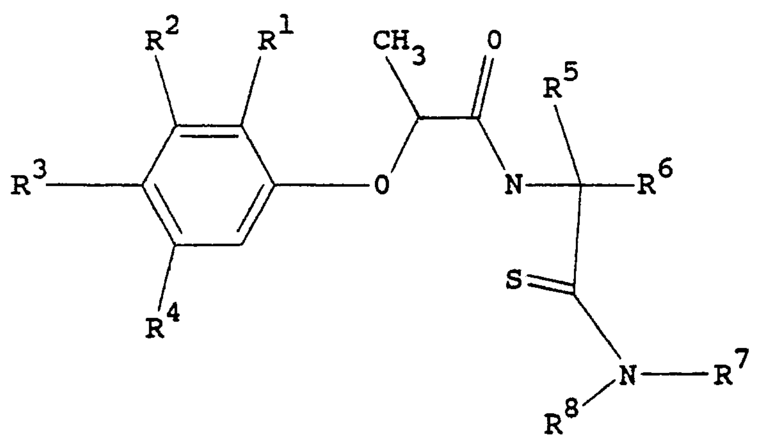

(I)

is prepared and used as a fungicide.

EP 0 437 640 A1

# FUNGICIDAL AMINO-THIOCARBOXYLIC ACID AMIDES

The present invention relates to novel N-phenoxypropionyl amino-thiocarboxylic acid amides, their preparation, their use for the control of phytopathogenic fungi and to compositions comprising these compounds.

Phenoxypropionic acids are known to be herbicides. However, as disclosed in EPA 87112370, when they are acyl moieties of acylated amino carboxylic acid derivatives these compounds are fungicidally active. We have found now that substituted n-phenoxypropionyl amino-thiocarboxylic acid amides surprisingly show an excellent fungicidal activity, in particular against phytopathogenic fungi in rice.

Accordingly, the present invention provides a compound of general formula I

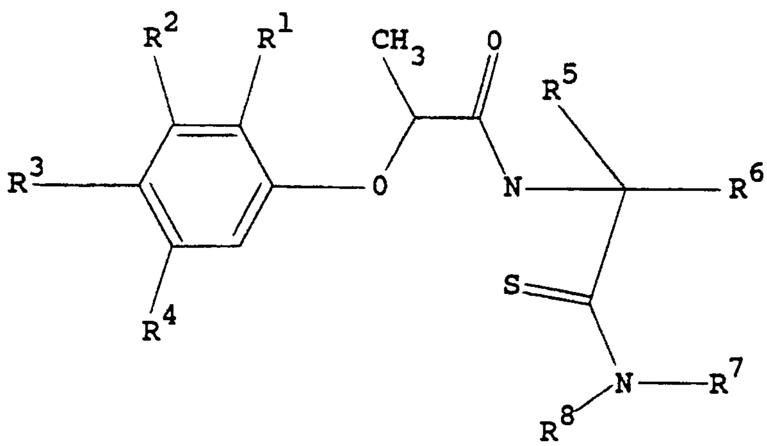

(I)

wherein

$R^1$, $R^2$ and $R^4$ are independently or simultaneously hydrogen, halogen or $C_1$-$C_6$-alkyl optionally substituted by one or more of the same or different halogen atoms,

$R^3$ is hydrogen, halogen, $C_1$-$C_6$-alkyl optionally substituted by one or more of the same or different halogen atoms, or phenoxy optionally substituted by one or more of the same or different halogen atoms, $R^5$ is hydrogen or $C_1$-$C_6$-alkyl optionally substituted by one or more of the same or different halogen atoms,

$R^6$ is hydrogen or $C_1$-$C_6$-alkyl optionally substituted by one or more of the same or different halogen atoms, and

$R^7$ and $R^8$ are independently or simultaneously hydrogen or $C_1$-$C_6$-alkyl.

Alkyl as substituent includes both straight chain or branched groups and, in the case of $R^1$-$R^5$, preferably contains 1-3 carbon atoms. Specific examples, depending on the number of carbon atoms include methyl, ethyl, propyl, butyl, pentyl, hexyl, etc. as well as their isomers such as isopropyl, isobutyl, tertiary-butyl, isopentyl. Halogen represents fluorine, chlorine, bromine, or iodine.

The compounds according to general formula I possess two chiral centres and, therefore, can form four stereoisomers. Their physical forms are oils, gums or crystalline solid materials at room temperature. They are superior by their valuable biocidal properties. For example, they can be used in agriculture or related fields for the control of phytopathogenic fungi such as Piricularia oryzae in rice. The compounds of general formula I according to the invention possess a high biocidal activity within a wide concentration range combined with an excellent selectivity and may be especially used in agriculture without any difficulties.

However, particularly good control of phytopathogenic fungi can be achieved with compounds of general formula I wherein $R^6$ is isopropyl and/or $R^7$ and $R^8$ are hydrogen.

The compounds according to general formula I may be prepared by a process known as such characterised in that an acid chloride of general formula II

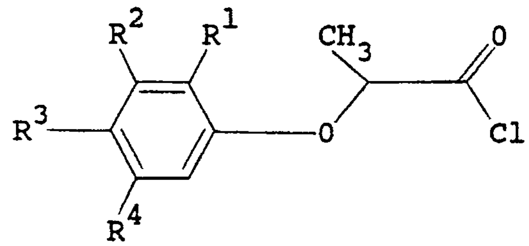

(II)

wherein $R^1$ to $R^4$ are as hereinbefore defined is reacted with an amino acid thioamide of general formula III

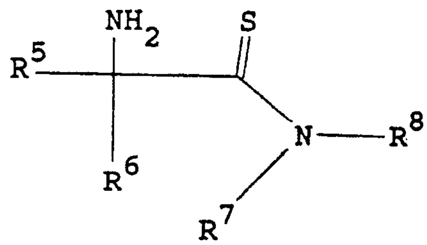

(III)

wherein $R^5$ to $R^8$ are as hereinbefore defined.

The starting material II may be prepared from appropriately substituted 2-phenoxypropionic acids by processes known in principle. Certain 2-phenoxypropionic acids are known as herbicides and are commercial products (R. Wegler, Chemie der Pflanzenschutz-und Schadlingsbekampfungsmittel, vol.2, Springer, Berlin, Heidelberg, New York, 1970). The acids may be reacted with a chlorinating agent such as thionyl chloride, phosphorous trichloride, phosphorous pentachloride, phosgene, etc., in inert solvents or without solvents yielding the respective acid chlorides according to general formula II. According to the reactivity of the components, the reaction may be carried out under cooling, at room temperature or at elevated temperature up to the boiling point of the reaction mixture. Generally the reaction takes place under reflux.

The amino thiocarboxylic acid amides III are known from literature (e.g. $R^5$, $R^7$, $R^8$ = H, $R^6$ = $-CH_2(CH_3)_2$: Can.J.Chem.65,282 (1987); $R^5$ = $CH_3$, $R^6$ = $-CH_2(CH_3)_2$, $R^7$ , $R^8$ = H: US 4,439,607). They are purified and stored as hydrochlorides.

The reaction of acid chlorides II with the amino-thiocarboxylic acid amides III is carried out in a way known in principle, whereby, if practicable, inert solvents which do not interfere with the reaction, e.g. toluene, benzene, diisopropyl ether, diglyme, tetrahydrofuran, or solvents which promote the reaction, e.g. pyridine, lutidine, are used. Advantageously, the hydrochloride of an amide according to general formula III is dissolved or suspended in an inert or promoting solvent, and a double equivalent of tertiary amine, e.g. triethylamine, diisopropyl ethylamine, 2,4,6-collidine, is added. Subsequently, one equivalent or an excess of the acid chloride II is added to the reaction mixture. However, another sequence of addition of the components is also in the scope of the invention. According to the reactivity of the components the reaction may be carried out under cooling, at room temperature or at elevated temperature up to the boiling point of the reaction mixture. Generally the reaction takes place at a temperature in the range of 10 °C to 100 °C.

Because the compounds according to general formula I possess two chiral centres, the preparation from racemic starting materials may yield up to four stereoisomers. The pure isomers may be obtained with optically pure educts.

The compounds according to general formula I may also be prepared by a process characterised in that an amino acid thioamide of general formula III wherein $R^5$ to $R^8$ are as hereinbefore defined is reacted with an acid anhydride of general formula IV

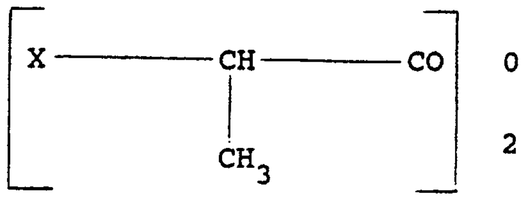

(IV)

wherein X is halogen
and the formed intermediate is reacted further with a compound of general formula V

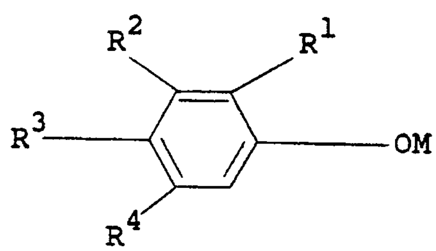

(V)

wherein $R^1$ to $R^4$ are as hereinbefore defined and M is an alkali metal.

The compounds according to formula I may be used for the control of phytopathogenic fungi. They are especially useful for the combating of fungal diseases in rice, particularly such caused by Piricularia oryzae. Although some of the novel compounds are derivatives of herbicides they surprisingly show an excellent plant tolerance.

The invention also provides fungicidal compositions which comprise at least one of the compounds according to general formula I as well as procedures for control of phytopathogenic fungi.

The compounds according to general formula I may be used as such, however, they are preferably used as compositions comprising, besides the compounds according to the invention, adjuvants and auxiliaries which are known for formulation purposes and are manufactured into e.g. emulsion concentrates, solutions which may be sprayed directly or diluted, diluted emulsions, wettable powders, soluble powders, dusts, granulates, microencapsulates by well-established procedures. The form of application such as spraying, atomising, dispersing, pouring may be chosen like the compositions according to the desired objectives and the given circumstances.

The formulations, i.e. the compositions which comprise at least one compound according to general formula I and optionally solid and/or liquid auxiliaries and adjuvants, may be prepared by well-established procedures, e.g. intensive mixing and/or grinding of the active ingredients with other substances, such as fillers, solvents, solid carriers, and optionally surface-active compounds (tensides).

Solvents may be aromatic hydrocarbons, preferably the fractions $C_8$ to $C_{12}$, e.g. xylenes or xylene mixtures, substituted naphthalenes, phthalic acid esters, such as dibutyl or dioctyl phthalate, aliphatic hydrocarbons, e.g. cyclohexane or paraffins, alcohols and glycols as well as their ethers and esters, e.g. ethanol, ethyleneglycol mono- and dimethyl ether, ketones such as cyclohexanone, strongly polar solvents such as N-methyl 2-pyrrolidone, dimethyl sulfoxide, alkyl formamides, epoxidized vegetable oils, e.g. epoxidized coconut or soybean oil, water.

Solid carriers, which may be used for dusts or dispersible powders, may be mineral fillers, such as calcite, talc, kaolin, montmorillonite, attapulgite. The physical properties may be improved by addition of highly dispersed silica gel or highly dispersed polymers. Carriers for granulates may be porous material, e.g. pumice, broken brick, sepiolite, bentonite, non-sorptive carriers may be calcite or sand. Additionally, a multitude of pre-granulated inorganic or organic materials may be used, such as dolomite or crushed plant residues.

Suitable surface-active substances may be non-ionogenic, anionic or cationic tensides with good dispersing, emulgating and wetting properties depending on the nature of the compound according to general formula I to be formulated. Tensides may also mean mixtures of tensides.

Suitable tensides may be so-called water-soluble soaps as well as water-soluble synthetic surface-active compounds.

Soaps usually are alkali, earth alkali or optionally-substituted ammonium salts of higher fatty acids ($C_{10}$-$C_{20}$), e.g. the sodium or potassium salts of oleic or stearic acid or of mixtures of natural fatty acids which are prepared, for example, from coconut or tallow oil. Furthermore, methyl-taurin salts of fatty acids may be used.

However, so-called synthetic tensides are preferably used, especially fatty sulphonates, fatty sulphates, sulphonated benzimidazole derivatives or alkyl aryl sulphonates.

The fatty sulphates or fatty sulphonates are normally used as alkali, earth alkali or optionally-substituted ammonium salts and have an alkyl moiety of 8 to 22 carbon atoms, whereby alkyl also means the alkyl moiety of acyl residues, such as the sodium or calcium salt of lignin sulphonic acid, of sulphuric acid dodecylate or of a mixture of fatty alcohols prepared from natural fatty acids. This also includes the salts of sulphuric acid esters, sulphonic acids and adducts of fatty alcohols and ethylene oxide. The sulphonated benzimidazole derivatives preferably contain 2 sulphonic acid residues and a fatty acid residue with 8 to 22 carbon atoms. Alkyl aryl sulphonates are, for example, the sodium, calcium or triethyl ammonium salts of dodecyl benzene sulphonic acid, dibutyl napthalene sulphonic acid or of a condensate of naphthalene sulphonic acid and formaldehyde.

Furthermore, phosphates, such as the salts of the phosphoric acid ester of a p-nonylphenol-(4-l4)-ethylene oxide adduct or phospholipids, may be used.

Non-ionic tensides are preferably polyglycolether derivatives of aliphatic or cycloaliphatic alcohols, saturated or non-saturated fatty acids and alkylphenols, which have 3 to 10 glycol ether groups and 8 to 20 carbon atoms in the (aliphatic) hydrocarbon residue and 6 to 18 carbon atoms in the alkyl residue of the alkyl phenols.

Other suitable non-ionic tensides are the water-soluble, 20 to 250 ethylene glycol ether groups containing polyadducts of ethylene oxide and polypropylene glycol, ethylene diamino polypropylene glycol and alkyl polypropylene glycol with 1 to 10 carbon atoms in the alkyl moiety, the substances normally contain 1 to 5 ethylene glycol units per propylene glycol unit.

Examples of non-ionic tensides are nonylphenol polyethoxy ethanols, castor oil polyglycol ether, polyadducts of ethylene oxide and polypropylene, tributyl phenoxy polyethoxy ehtanol, polyethylene glycol, octyl phenoxy polyethoxy ethanol.

Furthermore, fatty acid esters of polyoxy ethylene sorbitan such as polyoxy ethylene sorbitan trioleate may be used.

Cationic tensides preferably are quarternary ammonium salts which have at least one alkyl residue with 8 to 22 carbon atoms and, furthermore, low, optionally-halogenated alkyl, benzyl or hydroxyalkyl residues. The salts are preferably halides, methyl sulphates or alkyl sulphates, e.g. stearyl trimethyl ammonium chloride or benzyl bis(2-chloroethyl) ethyl ammonium bromide.

The tensides generally used for compositions are disclosed in publications as:
"McCutheon's Detergents and Emulsifiers Annual", MC Publishing Corp., Ridgewood, NJ, USA 1981;
H. Stache, "Tensid-Taschenbuch", 2nd ed., C. Hanser, Munich, Vienna, 1981;
M. and J. Ash, "Encyclopedia of Surfactants", vol. I-III, Chemical Publishing Co., New York, NY, USA 1980-1981.

The pesticidal compositions usually comprise 0.1% to 95%, preferably 0.1% to 80% of at least one compound according to general formula I, 1% to 99.9% of a solid or liquid adjuvant and 0% to 25%, preferably 0.1% to 25%, of a tenside.

The preferred compositions usually comprise:

Emulsion Concentrates:
Active Ingredient: 1% to 20%, preferably 5% to 10%
Surface-active substance:
5% to 30%, preferably 10% to 20%
Liquid carrier:
50% to 94%, preferably 70% to 85%

Suspension-Concentrates:
Active ingredient: 5% to 75%, preferably 10% to 50%
Water:
94% to 24%, preferably 88% to 30%
Surface-active substance:
1% to 40%, preferably 2% to 30%

Wettable Powder
Active ingredient: 0.5% to 90%, preferably 1% to 80%
Surface-active substance:
0.5% to 20%, preferably 1% to 15%
Solid carrier:
5% to 95%, preferably 15% to 99%

Dusts
Active ingredient: 0.1% to 10%, preferably 0.1% to 1%
Solid carrier:
99.9% to 90%, preferably 99.9% to 99%

Granulates:
Active ingredient: 0.5% to 30%, preferably 3% to 15%
Solid carrier:
99.5% to 70%, preferably 97% to 85%

As commodity the compositions may preferably be in a concentrated form whereas the end-user generally employs diluted compositions. The compositions may be diluted to a concentration of 0.001% of active ingredient (a.i.). The doses usually are in the range from 0.01 to 10 kg a.i./ha.

The compositions may also comprise other auxiliaries such as stabilizers, defoamers, viscosity controlling agents, thickeners, adhesives, fertilisers or other active ingredients to obtain special effects.

## EXAMPLES

### Example 1:

N-2-(4-chloro-2-methylphenoxy)propionyl]-2-amino-3--methyl-thiobutyric acid amide

2-(4-Chloro-2-methylphenoxy)-propionic acid chloride was prepared according to well-established procedures by reaction of 2-(4-Chloro-2-methylphenoxy)-propionicacid (mecoprop) with thionyl chloride (1.1 equiv.) under reflux for 2h. The crude product was evaporated in vacuo at 70 °C. The acid chloride was purified by distillation ($bp_{3-4}$ 113-118 °C), however, it may also be used as crude material.

2-Amino-3-methyl-thiobutyric acid hydrochloride (1.68g, 0.01mol) was suspended in benzene (40ml) and triethylamine (2.2ml) were added. Then 2-(4-chloro-2-methylphenoxy)-propionic acid chloride (2.53g, 0.01mol), dissolved in benzene (10ml) was added dropwise under stirring. The reaction mixture was stirred at room temperature, subsequently washed with water, diluted hydrochloric acid and saturated aqueous sodium hydrogen-carbonate. The organic layer was dried and evaporated. The residue crystallised upon trituration with diisopropyl ether was collected by vacuum filtration.N-[2-(4-chloro-2-methylphenoxy)-propionyl]-2-amino-3-methyl-thiobutyric acid amide was obtained as colourless microcrystalline material (mp. 130 °C).

$C_{15}H_{21}ClN_2O_2S$

| | | | | |
|---|---|---|---|---|
| Calcd. | C 55.00 | H 6.45 | N. 8.55 | S 9.75 % |
| Found | 54.77 | 6.54 | 8.51 | 9.86 % |

The compounds of Table 1 were prepared analogously to Example 1.

Table 1:

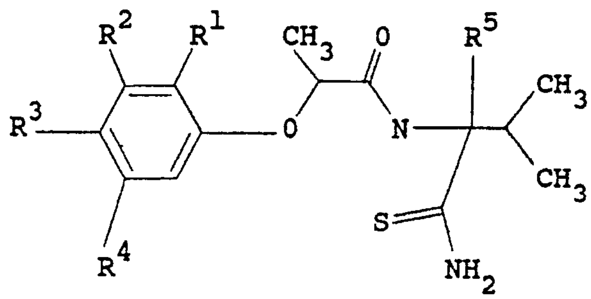

| No. | $R^1$ | $R^2$ | $R^3$ | $R^4$ | $R^5$ | mp. [°C] |
|---|---|---|---|---|---|---|
| 1 | Cl | H | Cl | H | H | 138 - 142 |
| 2 | $CH_3$ | H | Cl | H | $CH_3$ | 140 |
| 3 | Cl | H | Cl | H | $CH_3$ | oil |
| 4 | H | Cl | Cl | H | $CH_3$ | 155 |
| 5 | Cl | H | $CF_3$ | H | $CH_3$ | oil |
| 6 | H | H | 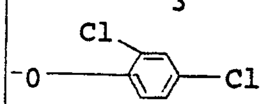 | H | $CH_3$ | oil |
| 7 | H | $CH_3$ | Cl | H | $CH_3$ | oil |
| 8 | H | Cl | H | Cl | $CH_3$ | 110-124 |

Example 2:

Formulation:

1. Emulsion concentrate:

| | |
|---|---|
| 5.0% (w/w) | active compound (a.i.) |
| 3.4% (w/w) | epoxidized vegetable oil |
| 13.4% (w/w) | emulgator combined of fatty alcohol glycol ether and calcium alkylaryl sulphonate |
| 40.0% (w/w) | N,N-dimethyl formamide |
| 38.2% (w/w) | xylenes |

The components were mixed and diluted with water to 0.01-0.1% (w/w) a.i. for application.

2. Wettable Powder:

| | |
|---|---|
| 10.0% (w/w) | active compound |
| 3.0% (w/w) | sodium fatty alcohol sulphonate |
| 5.0% (w/w) | salts of naphthalene sulphonic acid-formaldehyde condensate |
| 82.0% (w/w) | kaolin |

Example 3:

Biological Activity against Piricularia oryzae in rice

A. Leaf treatment:

Rice plants were pre-grown in plant-trays. They were sprayed to run-off with emulsions or suspensions which contained 1000, 500 or 250ppm of active compound. Two days after treatment, the trays were placed in the field between rice plants infected with *Piricularia oryzae* for 5-6 days in order to achieve an infection. The evaluation of the activity was carried out 5-8 days later using an evaluation scale from 1 to 3 (Table 2):

1: no infection
2: little infection
3: infection as in untreated control.

B. Soil treatment:

Rice plants were pre-grown in flower-pots. Emulsions or suspensions comprising 500ppm of active compound were poured onto the roots. Two days after treatment, the pots were placed in the field between rice plants infected with *Piricularia oryzae* for 4-6 days in order to achieve an infection. The evaluation of the activity was carried out 5-7 days later as described above (Table 2).

C. Treatment of submerged plants:

Rice plants were planted in buckets filled with soil. Water was filled in to shallow covering of the soil and an emulsion or suspension comprising the active ingredient (a.i.) was added in a dosage of 2,4 or 8 kg a.i./ha. Two days after treatment, the test-plants were placed in the field between rice plants infected with *Piricularia oryzae* and stayed there for the duration of the experiment. The evaluation was started the day after the disease could be observed with the untreated control and was carried out 4 - 5 times (3 tests with each substance). The evaluation scale was as described above, the figures in Table 2 are means of several assessments.

Table 2:

| Compound | Evaluation | | | | |
| --- | --- | --- | --- | --- | --- |
| | Concentr. [ppm] | A | B | Dosage [kg a.i/ha] | C |
| Example 1 | 1 000 | 1 | - | 8 | 3 |
| | 500 | 2 | 2 | 4 | - |
| | 250 | 3 | - | 2 | - |
| Table 1 no.1 | 1 000 | 1 | - | 8 | 3 |
| | 500 | 1 | 3 | 4 | 3 |
| | 250 | 1 | - | 2 | 3 |
| Table 1 no.2 | 1 000 | 1 | - | 8 | 1-2 |
| | 500 | 1-2 | 2 | 4 | 1-2 |
| | 250 | 1-2 | - | 2 | 2 |
| Table 1 no.3 | 1 000 | 1 | - | 8 | 1-2 |
| | 500 | 1-2 | 2 | 4 | 2 |
| | 250 | 1-2 | - | 2 | 2 |
| Table 1 no.4 | 1 000 | 1 | - | 8 | 3 |
| | 500 | - | - | 4 | - |
| | 250 | - | - | 2 | - |
| Table 1 no.5 | 1 000 | 1 | - | 8 | 1 |
| | 500 | 1-2 | 2-3 | 4 | - |
| | 250 | 2 | - | 2 | - |

## Claims

1. A compound of general formula I

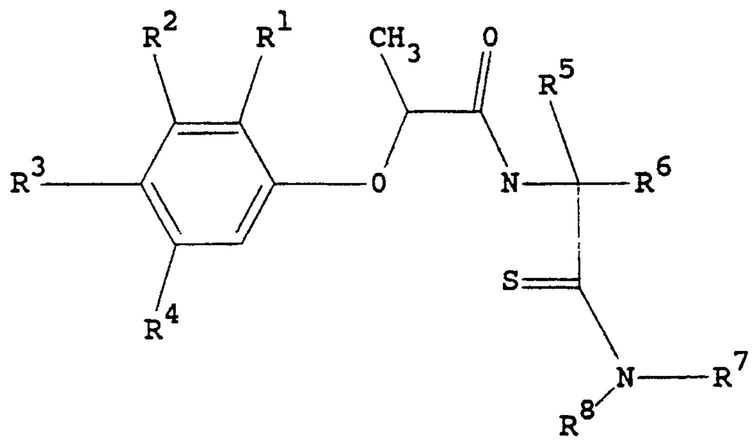

(I)

wherein

| | |
|---|---|
| $R^1$, $R^2$ and $R^4$ | are independently or simultaneously hydrogen, halogen or $C_1$-$C_6$-alkyl optionally substituted by one or more of the same or different halogen atoms, |
| $R^3$ | is hydrogen, halogen, $C_1$-$C_6$-alkyl optionally substituted by one or more of the same or different halogen atoms, or phenoxy optionally substituted by one or more of the same or different halogen atoms, |
| $R^5$ | is hydrogen or $C_1$-$C_6$-alkyl optionally substituted by one or more of the same or different halogen atoms, |
| $R^6$ | is hydrogen or $C_1$-$C_6$-alkyl optionally substituted by one or more of the same or different halogen atoms, and |
| $R^7$ and $R^8$ | are independently or simultaneously hydrogen or $C_1$-$C_6$-alkyl. |

**2.** A compound of general formula I as claimed in claim 1 wherein $R^1$, $R^2$ and $R^4$ each independently 3 represent hydrogen, chlorine or $C_{1-3}$ alkyl; $R^3$ represents hydrogen, chlorine or (halo) $C_{1-3}$ alkyl; $R^5$ represents hydrogen or $C_{1-3}$ alkyl; $R^6$ represents $C_{1-6}$ alkyl and $R^7$ and $R^8$ each represents hydrogen or $C_{1-3}$ alkyl.

**3.** A compound as claimed in claim 1 wherein $R^6$ is isopropyl.

**4.** A compound as claimed in claims 1, 2 or 3 wherein $R^7$ and $R^8$ both represent hydrogen.

**5.** A process for the preparation of a compound as defined in claim 1 characterised in that an acid chloride of general formula II

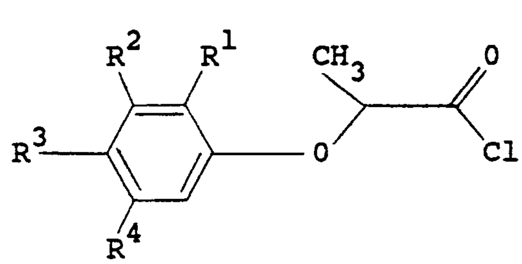

(II)

wherein $R^1$ to $R^4$ are as hereinbefore defined in Claim 1 is reacted with an amino acid thioamide of general formula III

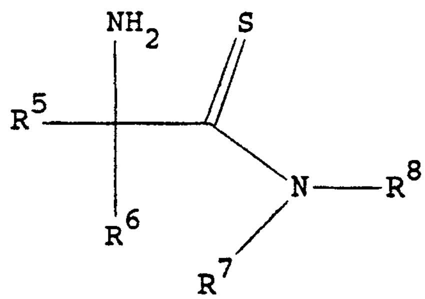

(III)

wherein $R^5$ to $R^8$ are as hereinbefore defined in Claim 1.

6. Use of a compound according to any one of Claims 1 to 4 for the control of phytopathogenic fungi.

7. Use of a compound according to any one of Claims 1 to 3 for the treatment of fungal diseases in rice.

8. Composition for the control of phytopathogenic fungi comprising at least one of the compounds according to any one of Claims 1 to 4 in association with a carrier and/or surface-active agent.

European Patent Office

# EUROPEAN SEARCH REPORT

Application Number

EP 90 10 0565

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF CHEMICAL SOCIETY no. 8, 1972, pages 478,479, Chemical Communications, Letchworth, UK; R. THOMAS: "Phenoxyacetylthioglycyl-(+-)-valine, a Fungal Metabolite of Anhydropenicilin V" * page 479 * | 1 | C 07 C 327/42<br>A 01 N 37/46 |
| A | PATENT ABSTRACTS OF JAPAN vol. 11, no. 184 (C-427)(2631), 12th June 1987; & JP - A - 62 005 944 (SUNTORY LTD) 12.01.1987 | 1,5 | |
| A | PATENT ABSTRACTS OF JAPAN vol. 11, no. 47 (C-403)(2494), 13th February 1987; & JP - A - 61 212 556 (MITSUI TOATSU CHEM.) 20.09.1986 | 1,6 | |
| A,D | US-A-4 439 607 (THOMAS W. DRABB et al.) * column 7, example 4 * | 5 | TECHNICAL FIELDS SEARCHED (Int. Cl.5) |
| A | EP-A-0 135 711 (AMERICAN CYANAMID) * pages 9,14,21,30, claim 1 * | 5 | C 07 C 327/00<br>A 01 N 37/00 |

The present search report has been drawn up for all claims

| Place of search | Date of completion of the search | Examiner |
|---|---|---|
| BERLIN | 04-04-1990 | RUFET J.M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document